# EUROPÄISCHE PATENTSCHRIFT

(11) **EP 2 803 654 B1**
(45) Veröffentlichungstag und Bekanntmachung des Hinweises auf die Patenterteilung: **30.11.2016**
(21) Anmeldenummer: 13168043.1
(22) Anmeldetag: 16.05.2013
(51) Int. Cl.: C07C 1/12, C07C 9/04, C07C 29/151, C07C 31/04, C10G 2/00, C10L 3/06, C12M 1/00, C12P 7/08, C25B 1/04

(54) **System und Verfahren zur Erzeugung von aliphatischen Alkoholen**
System and method for producing aliphatic alcohols
Système et procédé de production d'alcools aliphatiques

(43) Veröffentlichungstag der Anmeldung: 19.11.2014
(73) Patentinhaber: Schweitzer, Christian, 04357 Leipzig (DE); Hensel, Robert, 04416 Markkleeberg (DE)
(72) Erfinder: Schweitzer, Christian, 04357 Leipzig (DE); Hensel, Robert, 04416 Markkleeberg (DE)
(74) Vertreter: Kailuweit & Uhlemann Patentanwälte Partnerschaft mbB

(56) Entgegenhaltungen:
- WO-A1-2010/002469
- US-A- 5 342 702

## Beschreibung

Die Erfindung betrifft eine Vorrichtung und ein Verfahren zur Erzeugung von aliphatischen Alkoholen, insbesondere Bioethanol.

Bioethanol wird aus nachwachsenden Rohstoffen hergestellt und kommt im Kraftstoffsektor zum Einsatz. Bioethanol kann aus verschiedenen Pflanzen und pflanzlichen Teilen hergestellt werden: aus zuckerhaltigen Pflanzen wie Zuckerrüben und Zuckerrohr, aus stärkehaltigen Pflanzen wie Getreide, Kartoffeln und Mais, sowie aus zellulosehaltigen Rohstoffen wie z. B. Holz.

Zur Gewinnung von Bioethanol werden die in den Pflanzen enthaltenen Kohlenhydrate mit Hilfe von Enzymen oder Hefepilzen zu Alkohol vergoren. Für die Produktion von Bioethanol aus zucker- und stärkehaltige Pflanzen gibt es seit geraumer Zeit gängige Verfahren.

Damit Bioethanol-Kraftstoff einen sinnvollen Beitrag zur Energiewirtschaft leisten kann, muss die Herstellung eine positive Energiebilanz aufweisen. Den hauptsächlichen Anteil der aufgewendeten Primärenergie (Input) macht der nachwachsende und CO₂-neutrale Rohstoff Biomasse aus.

Bei der Fermentation der Rohstoffe und der Verbrennung des Bioethanols wird zwar das Treibhausgas Kohlenstoffdioxid freigesetzt; da jedoch beim Wachstum der Rohstoffpflanzen zuvor die gleiche Menge Kohlenstoffdioxid aus der Atmosphäre durch die Photosynthese gebunden wurde, sind diese chemischen Vorgänge (Photosynthese, Fermentation, Verbrennung) in der Addition CO₂-neutral. Da bei der Produktion der Rohstoffe und bei der Ethanolherstellung zusätzliche Energie benötigt wird, ist der Herstellungsprozess insgesamt nicht CO₂-neutral oder gar klimaneutral.

Aus den zur Ethanolgewinnung nicht benötigten Pflanzenbestandteilen wie Eiweiß, Pflanzenfasern und Fetten entstehen Nahrungs-, Futter- und Düngemittel. Getreideschlempe ist nährstoffreich und wird getrocknet als Futtermittel mit hohem Proteingehalt vermarktet (Trockenschlempe, auch DDGS = "dried distillers grains and solubles"). Bei der Herstellung von einem Liter Bioethanol aus Getreide entsteht so zusätzlich ein Kilogramm Proteinfutter. Vinasse, die bei der Melassevergärung zurückbleibt, wird agrartechnisch zum Beispiel ebenfalls als Tierfutterzusatz oder als Düngemittel genutzt.

Eine weitere Möglichkeit für die Verwendung der Schlempe ist die Energiegewinnung durch thermische Verwertung, d. h. die Verbrennung zwecks Dampferzeugung für die Ethanolanlage. Neben einer Senkung der Produktionskosten wird dadurch die Treibhausgasbilanz der Produktion verbessert. Energetisch interessant ist außerdem die Vergärung von Schlempe und anderen Reststoffen der Bioethanolproduktion in Biogasanlagen. Das gewonnene Biogas verbleibt als Prozesswärme in der Anlage oder wird ins Netz eingespeist. Es kann wie Erdgas als Energieträger in Haushalten oder auch als Kraftstoff genutzt werden.

Aus der DE 10 2007 015 623 A1 ist ein Verfahren zur energetischen Nutzung von landwirtschaftlich erzeugten Rohstoffen vorbekannt. Dabei wird der in einem Heizkraftwerk zum Betreiben einer Dampfturbine gebildete Prozessdampf für verfahrenstechnische nachgeordnete Anwendungen, wie etwa für eine Bioethanolanlage zur Erzeugung von Bioethanol verwendet, wobei für deren energetische Prozesse ein Teil der im Restdampf enthaltenen Energie nutzbar gemacht wird. Weiterhin kann der DE 10 2007 015 623 A1 entnommen werden, dass die bei der Bioethanolerzeugung anfallende Schlempe bei einer Biogaserzeugung verwendet werden kann. Diese Schlempe weist einen hohen Anteil organischer Stoffe auf. Daher ist selbige sehr gut für die Biogaserzeugung geeignet.

Weiterhin offenbart die DE 10 2007 001 614 A1 ein energieautarke Verfahren zur Herstellung von Bioethanol. Dabei wird die in einer Phasentrennung abgetrennte Dicksauermaische zum Zweck der Gewinnung von Biomethan für die Erzeugung von Prozessenergie in einem Blockheizkraftwerk einer parallel betriebenen Methanisierungsstufe zugeführt. Bei einer weiteren Variante des dortigen Verfahrens wird als Prozesswasser das Kondensat aus destilliertem Schlempewasser eingesetzt. Darüber hinaus soll sich das als Schlempewasser anfallende Sumpfprodukt auch wenigstens teilweise zur Gewinnung von Prozesswasser verwenden lassen, indem zur Gewinnung von salzarmem Prozesswasser und salzreichem Konzentrat aus dem Schlempewasser eine Vakuum-Umlaufverdampfungsanlage, eine Mikrofiltrations-, Ultrafiltrations-, Nanofiltrations- sowie Umkehrosmose- und/oder Membrantechniken eingesetzt werden.

Die DE 10 2010 005 818 A1 offenbart ein Verfahren bei dem eine direkte Rückführung des Ablaufs der Biogasanlage zum Zweck des Einmaischens in den Fermenter einer Bioethanolgewinnungsanlage derart vorgenommen wird, dass der Frischwassereinsatz bezogen auf den Gesamtprozess einer wesentlichen Reduzierung unterliegt. Dabei wird aus dem Ablauf der Biogasanlage ein Teilstrom ausgeschleust, mittels eines Membranverfahrens gereinigt und das erhaltene Permeat ganz oder teilweise zum Anmaischen in die Bioethanolgewinnungsanlage eingebracht.

Schließlich offenbart die DE 10 2008 058 501 B4 ein Verfahren zum Betreiben einer

Anlage zur Herstellung von Bioethanol, bei dem die organische Abfallprodukte des Herstellungsprozesses, verbrannt werden und die so gewonnene Nutzwärme der Anlage selbst wieder zugeführt wird. Die so gewonnene Nutzwärme wird in der Bioethanolgewinnungsanlage selbst wieder verbraucht, wobei sich viele Möglichkeiten finden, Wärme zu nutzen, sei dies nun, um Dampf herzustellen, oder sei dies, um direkt Anlagenteile oder Materialien zu erwärmen.

US 5,342,702 zeigt ein System, in dem in Fermentern Ethanol hergestellt und in den Fermentern erzeugtes Kohlenstoffdioxid mit Wasserstoff zu Methanol umgesetzt wird.

Trotz der aktuellen Fortschritte bei der Optimierung des Energiebedarfs zur Herstellung von Bioethanol, weisen die im bisherigen Stand der Technik bekannten Verfahren noch Potential zu einer verbesserten energieeffizienteren Prozessführung auf.

Die Aufgabe der vorliegenden Erfindung besteht daher darin, eine Vorrichtung und ein Verfahren anzugeben, welche die Nachteile des Stands der Technik überwinden.

Die Aufgabe wird durch eine Vorrichtung gemäß Anspruch 1 und ein Verfahren gemäß Anspruch 6 gelöst. Vorteilhafte Ausgestaltungen sind in den abhängigen Ansprüchen angegeben.

Erfindungsgemäß wird ein System zur Erzeugung von aliphatischen Alkoholen vorgeschlagen, umfassend:
- zumindest eine erste Vorrichtung zur biologischen Herstellung von Ethanol, wobei Kohlenstoffdioxid gebildet wird und
- zumindest eine zweite Vorrichtung zur chemischen Umsetzung des Kohlenstoffdioxids zu Methanol oder Methan,
wobei die zweite Vorrichtung zur chemischen Synthese von Methanol oder Methan der ersten Vorrichtung zur biologischen Herstellung des Ethanols nachgeschaltet ist.

Das erfindungsgemäße System weist eine zweite Verbindungsleitung von der zweiten Vorrichtung zur ersten Vorrichtung auf, die es ermöglicht, in der zweiten Vorrichtung gebildetes Wasser in die erste Vorrichtung einzuleiten. Das in der zweiten Vorrichtung gebildete Wasser wird dabei zum Anmaischen verwendet.

Weiter umfasst das erfindungsgemäße System zumindest eine Vorrichtung zur Dampferzeugung und eine dritte Verbindungsleitung, wobei die dritte Verbindungsleitung zwischen der Vorrichtung zur Dampferzeugung und der ersten Vorrichtung angeordnet ist und es ermöglicht, den in der Vorrichtung zur Dampferzeugung gebildeten Dampf in die erste Vorrichtung einzuleiten. Dabei wird der Vorrichtung zur Dampferzeugung beispielsweise chemische gebundene Energie in Form von Kohlenwasserstoffen zugeführt und mit Sauerstoff, beispielsweise aus vorbehandelter Umgebungsluft verbrannt. Beispielhaft soll an dieser Stelle die Oxidation von im Brennstoff enthaltenem reinen Kohlenstoff mit Sauerstoff stehen. Nach der Verbrennung entsteht ein stickoxidhaltiges Abgasgemisch. Die freie thermische Energie kann an vollentsalztes Wasser übertragen werden, wobei Wasserdampf entsteht, der als Übertragungsmedium für thermische und mechanische Energie in weitere Prozesse dienen kann.

Weiter weist das erfindungsgemäße System eine vierte Verbindungsleitung von der ersten Vorrichtung zur Vorrichtung zur Dampferzeugung auf, die es ermöglicht, ein in der ersten Vorrichtung gebildetes Kondensat in die Vorrichtung zur Dampferzeugung einzuleiten. Dabei wird in der ersten Vorrichtung die Energie aus dem Wasserdampf entnommen, wodurch dieser auskondensiert und als Kondensat über die vierte Verbindungsleitung dem Kreislauf zur Vorrichtung zur Dampferzeugung zurückgeführt wird. Damit steht der Vorrichtung zur Dampferzeugung Wasser aus dem Kondensat für eine erneute Aufnahme der thermischen Energie und Umwandlung in Wasserdampf zur Verfügung. Durch die Rückführung des Kondensats ist ein geringerer Wasserbedarf im Rahmen der Prozessführung durch Wiederverwendung und effektive Nutzung der Ausgangsstoffe gegeben.

Bevorzugt ist die erste Vorrichtung zur biologischen Herstellung von Ethanol durch Gärung ausgebildet. Im biochemischen Prozess zur Ethanolherstellung unter anoxischen Bedingungen wird aus Kohlenhydraten (hauptsächlich Glucose) Ethanol und Kohlenstoffdioxid erzeugt. Im Prozess wird die Fähigkeit von Mikroorganismen, bevorzugt Hefen, zur Energiegewinnung durch alkoholische Gärung genutzt, wenn die Umgebungsbedingungen für eine normale Zellatmung fehlen.

Bevorzugt ist die zweite Vorrichtung zur katalytischen Reduktion von Kohlenstoffdioxid mit Wasserstoff ausgebildet.

Bei der katalytischen Methanolsynthese wird aus Kohlenstoffdioxid und Wasserstoff Methanol und Wasser gewonnen. Der eingesetzt Katalysator ist in der Regel nicht besonders aktiv. Verbesserte Katalysatoreigenschaften werden durch Zuführung von mechanischer und thermischer Energie erzielt. Nach der Synthese steht Rohmethanol für die direkte Verbrennung zur Verfügung. Die Reaktion der Stoffumwandlung ist stark exotherm. Die thermische Energie kann dabei über die Umwandlung von Wasser zu Wasserdampf abgeleitet werden. Das in der Reaktion gebildete Wasser kann zudem im System zur Erzeugung von Wasserdampf oder zum Austransport von Prozessabwässern verwendet werden.

Weiter bevorzugt weist die erste Vorrichtung eine Vorrichtung zur Abtrennung von Ethanol auf. Das Ethanol wird dabei aus dem entstandenen alkoholhaltigen Gemisch mittels eines thermischen Trennverfahrens separiert und anschließend absolutiert. Dabei entstehen Prozessabwässer.

Weiter bevorzugt weist die zweite Vorrichtung eine Vorrichtung zur Abtrennung von Methanol auf. Die Abtrennung des Methanols kann dabei über eine destillative Aufarbeitung erfolgen. Hierzu kann die im Wasserdampf gespeicherte thermische Energie eingesetzt werden.

In einer weiteren Ausführungsform der Erfindung ist die zweite Vorrichtung zur chemischen Synthese von Methan ausgebildet. Bei der Methansynthese wird im Sabatier-Prozess katalytisch aus Kohlenstoffdioxid und Wasserstoff, Methan und Wasser gewonnen. Die Reaktionsformel lautet CO₂ + 4 H₂ → CH₄ + 2 H₂O. Die Reaktion der Stoffumwandlung ist stark exotherm. Die thermische Energie wird über die Umwandlung des entstehenden Wassers zu Wasserdampf abgeleitet.

Bevorzugt weist das erfindungsgemäße System eine erste Verbindungsleitung von der ersten Vorrichtung zur zweiten Vorrichtung auf, die es ermöglicht, ein in der ersten Vorrichtung gebildetes Kohlenstoffdioxid in die zweite Vorrichtung einzuleiten. Für die Methanol- oder Methansynthese stehen damit ökologisch unbedenklicher Wasserstoff und biogenes Kohlenstoffdioxid aus der ersten Vorrichtung zur Verfügung. Im Gegensatz zur Einzelanwendung kann auf diese Art ein grünes "biogenes" Methanol oder Methan erzeugt werden.

Weiter bevorzugt weist die Vorrichtung zur Dampferzeugung eine Vorrichtung zur Abtrennung von Stickoxiden auf. Die bei der Verbrennung der Kohlenwasserstoffe und Sauerstoff, welcher beispielsweise in Form der Umgebungsluft zugeführt wird, entstehenden Stickoxide werden dabei als Abgas abgetrennt.

Weiter bevorzugt umfasst das erfindungsgemäße System weiterhin zumindest eine Vorrichtung zur Generation von Wasserstoff, welche eine fünfte Verbindungsleitung zur zweiten Vorrichtung aufweist, die es ermöglicht, den Wasserstoff in die zweite Vorrichtung einzubringen. Beispielsweise ist dadurch die Zuführung von Wasserstoff zur chemischen Synthese von Methanol möglich.

Weiter bevorzugt ist die Vorrichtung zur Generation von Wasserstoff als Elektrolysevorrichtung ausgebildet.

Dabei ist die Elektrolysevorrichtung bevorzugt zur elektrolytischen Erzeugung von Wasserstoff und Sauerstoff aus Wasser ausgebildet. Das hierfür eingesetzte Wasser kann dabei beispielsweise als Kondensat aus der ersten Vorrichtung oder als Produkt aus der zweiten Vorrichtung stammen. Durch die Einspeisung des in den anderen Prozessen gebildeten Wassers in die Elektrolysevorrichtung und dessen Nutzung zur Herstellung von Wasserstoff ist wiederum eine optimale Nutzung der eingesetzten Ausgangsstoffe innerhalb des Systems möglich.

Weiter bevorzugt ist die Elektrolysevorrichtung als alkalische Elektrolysevorrichtung oder PEM-Elektrolysevorrichtung ausgebildet. Die alkalische Elektrolyse nutzt modulare Stapel von Elektrolysezellen. Die Elektroden sind bevorzugt aus perforierten Stahlblechen mit einer möglichst porösen Oberfläche ausgebildet. Die Elektroden sind bevorzugt als Vorbleche nahe an dem Diaphragma positioniert und bevorzugt elektrisch leitend mit den Endplatten (Einzelzelle) bzw. den bipolaren Trennblechen (Zellstapel) verbunden. Zellrahmen dichten die Halbzellen bevorzugt nach außen ab und dienen als Einbettung für das Diaphragma. Die Stromquelle wird über die Endplatten kontaktiert. Beide Halbzellen sind bevorzugt mit einem alkalischen Elektrolyten geflutet bzw. werden von dieser Lauge durchströmt. Bevorratet wird die Lauge bevorzugt in separaten Tanks, die gleichzeitig als Gas-Flüssig-Separator dienen. Die PEM-Elektrolysezelle hingegen umfasst eine Anode (Sauerstoffproduktion) und eine Kathode (Wasserstoff-Produktion), welche durch eine saure Protonaustauschmembran (PEM, engl.: proton exchange membrane) voneinander getrennt sind.

Weiter bevorzugt weist das erfindungsgemäße System eine sechste Verbindungsleitung auf, die es ermöglicht, den in der Elektrolysevorrichtung gebildeten Sauerstoff in die Vorrichtung zur Dampferzeugung einzuleiten. Dadurch ist eine Reduktion des Anteils an durch Umgebungsluft eingebrachten Sauerstoff möglich, was zudem zu einer Minimierung der Bildung von Stickoxiden führt.

Alternativ wird der Wasserstoff mittels biologischer Verfahren, z. B. durch Algen oder Cyanobakterien, hergestellt. In diesem Fall ist die Vorrichtung zur Generation von Wasserstoff bevorzugt ein Photobioreaktor, welcher Algen oder Cyanobakterien, enthält.

Gegenstand der Erfindung ist auch ein Verfahren zur Herstellung von aliphatischen Alkoholen, umfassend die Schritte:
- Herstellung eines ersten aliphatischen Alkohols mittels eines biologischen Verfahrens, wobei Kohlenstoffdioxid gebildet wird,
- Umsetzung des gebildeten Kohlenstoffdioxids zu Methanol oder Methan mittels einer chemischen, katalytischer Synthese.

Die Umsetzung des Kohlenstoffdioxids ist der Herstellung des ersten aliphatischen Alkohols nachgeschaltet und örtlich von dieser getrennt. Unter örtlich getrennt wird dabei in unterschiedlichen Reaktionsbehältern verstanden, die jedoch durch Verbindungsleitungen miteinander verbunden sein können. Die Herstellung des ersten aliphatischen Alkohols und die Umsetzung des Kohlenstoffdioxids sind derart verbunden, dass das Kohlenstoffdioxid als Produkt der Herstellung des ersten aliphatischen Alkohols zumindest teilweise als Edukt zur Herstellung eines Methanols oder Methans verwendet wird.

Erfindungsgemäß ist der erste aliphatische Alkohol Ethanol.

Bevorzugt erfolgt die Herstellung des ersten aliphatischen Alkohols durch Gärung mittels Mikroorganismen, wobei das in der Gärung gebildete Kohlenstoffdioxid als Edukt für die Herstellung des zweiten aliphatischen Alkohols verwendet wird.

Dabei erfolgt die Umsetzung des Kohlenstoffdioxids zu einem Kohlenwasserstoff mittels an sich bekannten chemischen Synthesen in Gegenwart von Katalysatoren und Wasserstoff zu substituierten (bevorzugt sauerstoffhaltigen) oder unsubstituierten Kohlenwasserstoffen. Dabei wird bevorzugt als Zwischenschritt Synthesegas gebildet. Die Zusammensetzung des gebildeten Kohlenwasserstoffs wird dabei in bekannter Weise durch die Auswahl der Katalysatoren, der Wasserstoffkonzentration und die Reaktionsbedingungen gesteuert.

In einer Ausführungsform der Erfindung wird als Kohlenwasserstoff Methanol gebildet.

Bevorzugt erfolgt die Herstellung des Methanols durch eine katalytische Synthese von Wasserstoff und Kohlenstoffdioxid. Dabei wird Methanol und Wasser gebildet. Das Wasser kann nachfolgend beispielsweise für die Herstellung des ersten aliphatischen Alkohols verwendet werden.

Vorteilhaft wird bei der katalytischen Synthese von Methanol exotherme Energie frei, welche bevorzugt über die Umwandlung von Wasser zu Wasserdampf abgeleitet wird. Die in Form von Wasserdampf gespeicherte thermische Energie kann vorteilhaft nachfolgend etwa zur Herstellung des ersten aliphatischen Alkohols, beispielsweise zum Anmaischen, genutzt werden. Dadurch ergibt sich eine effiziente Energienutzung innerhalb des Systems. Denkbar und bevorzugt ist auch die Nutzung der thermischen Energie für die Abtrennung des ersten oder zweiten aliphatischen Alkohols.

In einer weiteren Ausführungsform der Erfindung erfolgt die Umsetzung des Kohlenstoffdioxids mittels chemischer Synthese, wobei als Kohlenwasserstoff Methan gebildet wird. Nach der Synthese steht Methan für die direkte Verbrennung bzw. als chemischer Grundstoff zur Verfügung. Methan ist ein wichtiges Ausgangsprodukt für technische Synthesen von Wasserstoff, Methanol, Ethin, Blausäure, Schwefelkohlenstoff und Methylhalogeniden. Es dient als Ausgangspunkt für viele andere organische Verbindungen.

Erfindungsgemäß umfasst das Verfahren weiterhin ein Verfahren zur Dampferzeugung, welches derart mit der Herstellung des ersten aliphatischen Alkohols verbunden ist, dass der im Verfahren zur Dampferzeugung generierte Dampf zum Eintrag thermischer Energie für die Herstellung des ersten aliphatischen Alkohols verwendet wird. Der bei der Dampferzeugung generierte Dampf, wird dabei beispielsweise aus Wasser gebildet, welches über die Verbrennung kohlenstoffhaltiger Verbindungen mit Sauerstoff verdampft wird. Die in Form des Wasserdampfs gespeicherte thermische Energie wird nunmehr bevorzugt zum Anmaischen, zur Temperierung bei der Herstellung des ersten aliphatischen Alkohols und/oder für die Abtrennung des ersten und/oder zweiten aliphatischen Alkohols eingesetzt. Durch die Generation von Dampf steht ein als Übertragungsmedium für thermische und mechanische Energie zur Verfügung, welches für weitere Prozesse im System genutzt werden kann.

Weiter bevorzugt umfasst das Verfahren zur Dampferzeugung weiterhin die Abtrennung von Stickoxiden. Bei der Verbrennung von Sauerstoff, welcher beispielsweise in Form von Umgebungsluft zur Verbrennung eingesetzt wird, entstehen dabei Stickoxidverbindungen. Diese können zur Vermeidung des Einbringens in das System abgetrennt und als Abluft ausgetragen werden.

Weiter bevorzugt umfasst das Verfahren weiterhin ein Verfahren zur Generation von Wasserstoff, welches derart mit der Herstellung des zweiten aliphatischen Alkohols verbunden ist, dass der generierte Wasserstoff als Edukt für die chemische Synthese des zweiten aliphatischen Alkohols dient. Dabei ist das Verfahren zur Generation von Wasserstoff ein auf Elektrolyse basierendes Verfahren. Weiter ist das Verfahren zur Generation von Wasserstoff ein auf Elektrolyse basierendes Verfahren ausgewählt aus alkalischer Elektrolyse und PEM-Elektrolyse.

Die alkalische Elektrolyse nutzt bevorzugt modulare Stapel von Elektrolysezellen. Die Elektroden sind bevorzugt aus perforierten Stahlblechen mit einer möglichst porösen Oberfläche ausgebildet. Die Elektroden sind bevorzugt als Vorbleche nahe an dem Diaphragma positioniert und elektrisch leitend mit den Endplatten (Einzelzelle) bzw. den bipolaren Trennblechen (Zellstapel) verbunden. Zellrahmen dichten bevorzugt die Halbzellen nach außen ab und dienen als Einbettung für das Diaphragma. Die Stromquelle wird bevorzugt über die Endplatten kontaktiert. Beide Halbzellen sind bevorzugt mit einem alkalischen Elektrolyten geflutet bzw. werden von dieser Lauge durchströmt. Bevorratet wird die Lauge in separaten Tanks, die gleichzeitig als Gas-Flüssig-Separator dienen.

Die PEM-Elektrolysezelle hingegen umfasst eine Anode (Sauerstoffproduktion) und einer Kathode (Wasserstoff-Produktion), welche durch eine saure Protonaustauschmembran (PEM, engl.: proton exchange membrane) voneinander getrennt sind.

Weiter ist das Verfahren zur Generation von Wasserstoff ein auf der Elektrolyse von Wasser basierendes Verfahren. Dabei wird neben Wasserstoff durch die Elektrolyse auch Sauerstoff gebildet. Dieser Sauerstoff wird dabei für das Verfahren zur Dampferzeugung genutzt werden, wodurch der Anteil an Sauerstoff, welcher in Form von Umgebungsluft zur Verbrennung genutzt wird, minimiert oder vollständig ersetzt wird. Dieser Sauerstoff kann je nach Auslegung Luft als Oxidationsmittel ganz oder teilweise ersetzen. Dadurch verringert sich der Inertgasanteil. Der Energiebedarf zum Aufheizen dieser Bestandteile bis auf Zündtemperatur des Brennstoffes entfällt. Der Brennstoffbedarf sinkt. Die Effizienz der Dampferzeugung wird gesteigert. Ein weiterer Effekt zeigt sich durch die Begrenzung des Stickstoffeintrages. Der eingetragene Stickstoffanteil für jedes aus der Elektrolyse eingebrachte mol Sauerstoff sinkt von 3,76 mol bis maximal Null. Im Verbrennungsabgas sinkt der Stickoxidanteil deutlich.

Alternativ wird der Wasserstoff mittels biologischer Verfahren, bevorzugt durch Algen oder Cyanobakterien, hergestellt.

Weiter bevorzugt wird das bei der Herstellung des zweiten aliphatischen Alkohols gebildete Wasser für das Verfahren zur Generation von Wasserstoff eingesetzt. Dadurch kann der Bedarf an Wasser für die Elektrolyse verringert werden.

Weiter bevorzugt werden alle im Verfahren anfallenden Abwasserströme über die Herstellung des ersten aliphatischen Alkohols gesammelt. Dadurch können die Abwasserströme ökonomischer verbracht werden.

Das erfindungsgemäße Verfahren zeichnet sich durch die Kopplung eines ersten biologischen Verfahrens für die Herstellung eines ersten aliphatischen Alkohols und eines zweiten chemisch-synthetischen Verfahrens für die Herstellung von Methanol oder Methan aus, wobei CO₂ als Produkt des ersten Herstellungsverfahrens als Edukt für das zweite Herstellungsverfahren zumindest teilweise verwendet wird. Weiter umfasst das Verfahren daneben noch ein Verfahren zur Dampferzeugung, wobei der generierte Dampf als Übertragungsmedium für thermische und mechanische Energie für das Verfahren dient. Zudem kann das Verfahren vorteilhaft auch ein Verfahren zur Generation von Wasserstoff umfassen, welcher als Edukt zur chemischen Synthese des zweiten aliphatischen Alkohols dienen kann.

Für die Realisierung der Erfindung ist es auch zweckmäßig die vorgenannten bevorzugten Ausgestaltungen und Ausführungsformen miteinander zu kombinieren. Bevorzugte Weiterbildungen der Erfindung ergeben sich aus den Kombinationen der Ansprüche oder einzelner Merkmale davon.

Nachfolgend soll die Erfindung anhand einiger Ausführungsbeispiele und zugehöriger Figuren eingehender erläutert werden. Die Ausführungsbeispiele sollen dabei die Erfindung beschrieben ohne diese zu beschränken.

Es zeigen die
**Fig. 1** eine schematische Darstellung eines erfindungsgemäßen Systems gemäß einer ersten Ausgestaltung,
**Fig. 2** eine schematische Darstellung eines erfindungsgemäßen Systems gemäß einer zweiten Ausgestaltung und
**Fig. 3** eine schematische Darstellung eines erfindungsgemäßen Systems gemäß einer dritten Ausgestaltung.

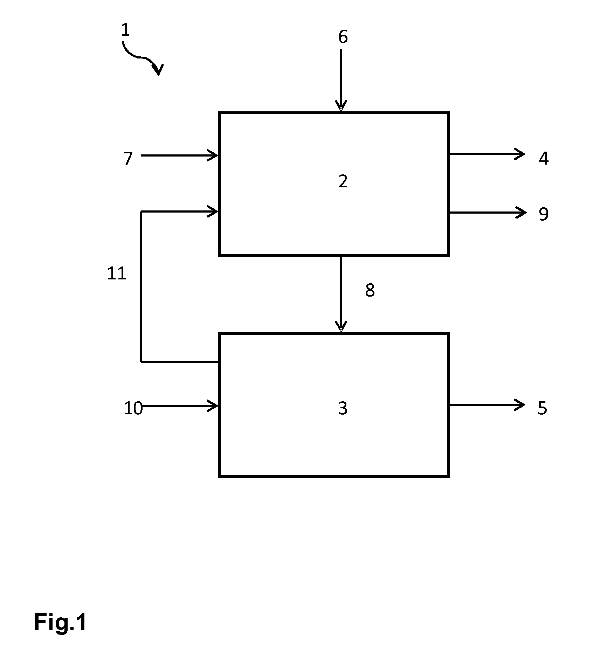

In der Fig. 1 ist beispielhaft ein erfindungsgemäßes System **1** gemäß einer ersten Ausgestaltung dargestellt. Das erfindungsgemäße System **1** umfasst eine Vorrichtung **2** zur biologischen Herstellung eines ersten aliphatischen Alkohols **4** sowie eine Vorrichtung **3** zur chemischen Synthese eines Kohlenwasserstoffs **5,** wie etwa eines zweiten aliphatischen Alkohols. Der erste aliphatische Alkohol **4** ist dabei Ethanol und der zweite aliphatische Alkohol **5** Methanol. Dabei kann das Ethanol **4** mittels alkoholischer Gärung durch Mikroorganismen gebildet werden. Hierzu werden Kohlenhydrate **6,** wie etwa Glucose, unter geeigneten Bedingungen und Wasser **7** in die Vorrichtung **2** eingebracht. Aufgrund der alkoholischen Gärung bildet sich neben Ethanol **4** auch Kohlenstoffdioxid **8.** Dieses Kohlenstoffdioxid **8** wird erfindungsgemäß für die chemische Synthese des Methanols **5** in der zweiten Vorrichtung **3** eingesetzt. Für die Synthese des Methanols **5** wird neben Kohlenstoffdioxid **8** auch Wasserstoff in die Vorrichtung **3** eingebracht und katalytisch zu Methanol **5** und Wasser **11** umgesetzt. Das gebildete Methanol **5** kann danach mittels einer nicht näher dargestellten Vorrichtung abgetrennt werden. Dies kann beispielsweise durch Destillation erfolgen. Das Wasser **11** wird nachfolgend für die alkoholische Gärung in die erste Vorrichtung **2** eingebracht und somit genutzt. Daneben fungiert das Wasser **11** auch als Energieträger, da es die bei der exothermen Reaktion der Methanolsynthese freigesetzte thermische Energie aufgenommen hat. Diese thermische Energie kann nun wiederum für die alkoholische Gärung in der ersten Vorrichtung **2** genutzt werden.

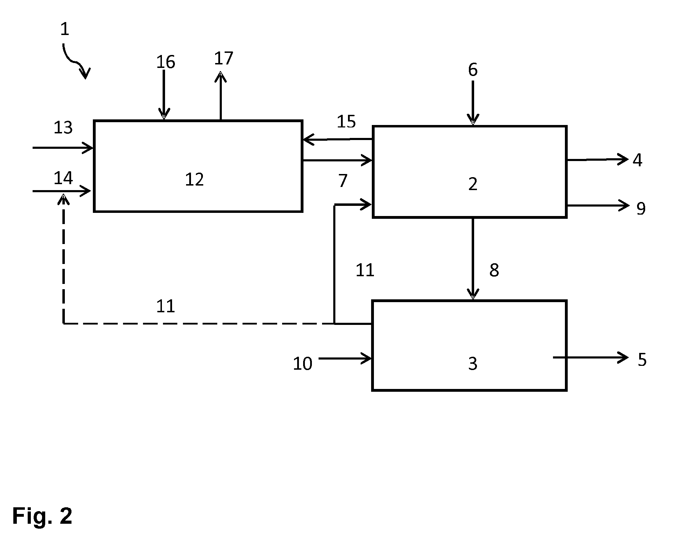

In einer weiteren Ausführungsform ist in der Fig. 2 beispielhaft ein erfindungsgemäßes System **1** gemäß einer zweiten Ausgestaltung dargestellt. Das System **1** entspricht dem vorher beschriebenen, wobei das System **1** nunmehr eine Vorrichtung zur Dampferzeugung **12** aufweist. In der Vorrichtung zur Dampferzeugung **12** werden kohlenstoffhaltige Verbindungen **16** unter Sauerstoff-Zufuhr **13** verbrannt. Der Sauerstoff **13** kann dabei beispielswiese als Umgebungsluft der Vorrichtung zur Verdampfung **12** zugeführt werden. Bei der Verbrennung entstehen Stickoxide **17,** welche abgetrennt und separat abgegeben werden. Die dabei entstehende thermische Energie wird auf das, der Vorrichtung **12** zugeführte Wasser **14** übertragen, wodurch diese verdampft und als Wasserdampf **7** der ersten Vorrichtung **2** zugeführt wird. Die im Wasserdampf enthaltene thermische Energie wird in der Vorrichtung **2** beispielsweise zur Temperierung oder zum Anmaischen verwendet, wobei dem Wasserdampf **7** beispielsweise über Wärmetauscher die thermische Energie entzogen wird. Infolgedessen kommt es zur Kondensation und das so gebildete Kondensat **15** wird dem Dampferzeuger zur erneuten Verdampfung zugeführt. Gleichfalls ist es auch denkbar, das in der zweiten Vorrichtung **3** bei der Methanolsynthese gebildete Wasser **11** der Vorrichtung zur Dampferzeugung **12** zuzuführen.

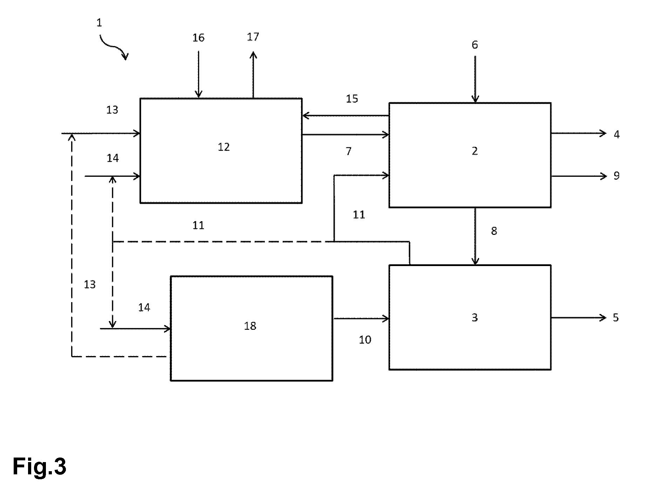

In einer weiteren Ausführungsform ist in der Fig. 3 beispielhaft ein erfindungsgemäßes System **1** gemäß einer dritten Ausgestaltung dargestellt. Das System **1** entspricht dem vorher beschriebenen, wobei das System zudem eine Vorrichtung zur Generation von Wasserstoff **10** umfasst. Dadurch muss der Wasserstoff **10** der zweiten Vorrichtung nicht mehr separat zugeführt werden, sondern kann im System **1** generiert werden. Der Wasserstoff **10** wird dabei beispielsweise mittels Elektrolyse von Wasser **14** gebildet, wodurch neben Wasserstoff **10** auch Sauerstoff **13** gebildet wird. Dieser Sauerstoff **13** kann für die Verbrennung der kohlenstoffhaltigen Verbindungen **16** in der Vorrichtung zur Dampferzeugung **12** genutzt werden. Dadurch verringert sich der Inertgasanteil. Der Energiebedarf zum Aufheizen dieser Bestandteile bis auf Zündtemperatur des Brennstoffes entfällt, weshalb der Brennstoffbedarf sinkt. Die Effizienz der Dampferzeugung wird dadurch gesteigert. Ein weiterer Effekt zeigt sich durch die Begrenzung des Stickstoffeintrages. Der eingetragene Stickstoffanteil für jedes aus der Elektrolyse eingebrachte mol Sauerstoff sinkt von 3,76 mol bis maximal Null. Im Verbrennungsabgas sinkt der Stickoxideanteil deutlich. Zudem kann das bei der Methanolsynthese gebildete Wasser **11** für die Elektrolyse in der Vorrichtung zur Wasserstoffgeneration **16** eingesetzt werden.

In einem weiteren nicht näher dargestellten Ausführungsbeispiel ist die Vorrichtung **3** zur chemischen Synthese eines Kohlenwasserstoffs **5** zur Bildung von Methan ausgebildet. Bei den Methansynthesen wird im Sabatier-Prozess katalytisch aus Kohlenstoffdioxid **8** und Wasserstoff **10,** Methan **5** und Wasser **11** gewonnen. Die Reaktionsformel lautet: CO₂ + 4 H₂ → CH₄ + 2 H₂O.

In einem weiteren Ausführungsbeispiel sind die theoretischen Stoffströme des erfindungsgemäßen Systems **1** bei der Bildung von Ethanol und Methanol berechnet worden.

Die folgende stöchiometrische Berechnung stellt ein theoretisches Verhältnis der Stoffströme zueinander auf Basis Einsatzgröße von 1.000 kg Kohlenstoffdioxid dar.

### Beispiel Dampferzeugung

| Eingang | | Ausgang | |
|---|---|---|---|
| Sauerstoff [g] | 1.090 kg | Kohlenstoffdioxid [g] | 1.499 kg |
| Kohlenstoff [s] | 409 kg | | |
| | 1.499 kg | | 1.499 kg |

### Beispiel Elektrolyse

| Eingang | | Ausgang | |
|---|---|---|---|
| Wasser [I] | 1.227 kg | Wasserstoff [g] | 137 kg |
| | | Sauerstoff [g] | 1.090 kg |
| | 1.227 kg | | 1.227 kg |

### Beispiel Ethanolherstellung

| Eingang | | Ausgang | |
|---|---|---|---|
| Glucose [s] | 2.046 kg | Kohlenstoffdioxid [g] | 1.000 kg |
| | | Ethylalkohol [I] | 1.046 kg |
| | 2.046 kg | | 2.046 kg |

### Beispiel Methanolsynthese

| Eingang | | Ausgang | |
|---|---|---|---|
| Kohlenstoffdioxid [g] | 1.000 kg | Wasser [g] | 409 kg |
| Wasserstoff [g] | 137 kg | Methylalkohol [I] | 728 kg |
| | 1.137 kg | | 1.137 kg |

In einem weiteren Ausführungsbeispiel sind die berechneten Stoffströme im System für die Bildung von Ethanol und Methanol in der Tab.1 dargestellt. Die Abkürzung s steht für fest (solid), f für gasförmig und I für flüssig (liquid).

**Tab.1**

| Nr. | | Summenformel | Mittlere Molare Masse | Aggregatzustand | H₂ | N₂ | O₂ | Ar | CO₂ | C₆H₅OH | CH₄O | C₂H₆O | H₂O | sonst. |
|---|---|---|---|---|---|---|---|---|---|---|---|---|---|---|
| | | | [g/mol] | | [mol %] | [mol %] | [mol %] | [mol %] | [mol %] | [mol %] | [mol %] | [mol %] | [mol %] | [mol %] |
| 01 | Kohlenstoff, rein | C | 12,01 | s | --- | --- | --- | --- | --- | --- | --- | --- | --- | 100 |
| 02 | Luft, trocken | N₂, O₂, CO₂,... | 28,95 | g | | 78,08 | 20,94 | 0,93 | 0,03 | --- | --- | --- | --- | 0,002 |
| 03 | Wasser, vollentsalzt | H₂O | 18,02 | l | --- | --- | --- | --- | --- | --- | --- | --- | 100 | --- |
| 04 | Kohlenstoffdioxid | CO₂ | 44,01 | g | --- | --- | --- | --- | 100 | --- | --- | --- | --- | --- |
| 05 | Wasser | H₂O | 18,02 | l | --- | --- | --- | --- | --- | --- | --- | --- | 100 | --- |
| 06 | Wasser | H₂O | 18,02 | g | --- | --- | --- | --- | --- | --- | --- | --- | 100 | --- |
| 07 | Sauerstoff | O₂ | 15,99 | g | --- | --- | 100 | --- | --- | --- | --- | --- | --- | --- |
| 08 | Wasser | H₂O | 18,02 | l | --- | --- | --- | --- | --- | --- | --- | --- | 100 | --- |
| 09 | Wasserstoff | H₂ | 1,01 | g | 100 | --- | --- | --- | --- | --- | --- | --- | --- | --- |
| 10 | Glucose | C₆H₁₂O₆ | 180,15 | s | --- | --- | --- | --- | --- | 100 | --- | --- | --- | --- |
| 11 | Kohlenstoffdioxid | CO₂ | 44,01 | | --- | --- | --- | --- | 100 | --- | --- | --- | --- | --- |
| 12 | Ethylalkohol | C2H6O | 46,07 | l | --- | --- | --- | --- | --- | --- | --- | 99,95 | --- | 0,05 |
| 13 | Abwasser | H2O | 18,02 | l | --- | --- | --- | --- | --- | --- | --- | --- | 70 | 30 |
| 14 | Wasser | H2O | 18,02 | g | --- | --- | --- | --- | --- | --- | --- | --- | 100 | --- |
| 15 | Methylalkohol | CH4O | 32,04 | l | --- | --- | --- | --- | --- | --- | 99,95 | --- | --- | 0,05 |
| 16 | Luft- SauerstoffGemisch | N2, O2 CO2,... | 28,95 | g | | < 78,08 | > 20,94 | <0,93 | <0,03 | --- | --- | --- | --- | < 0,002 |
| 17 | Wasser, vollentsalzt | H2O | 18,02 | l | --- | --- | --- | --- | --- | --- | --- | --- | 100 | --- |

In einem weiteren Ausführungsbeispiel sind die theoretischen Stoffströme des erfindungsgemäßen Systems 1 bei der Bildung von Ethanol und Methan berechnet worden.

Die theoretische Umsetzung der Massenverhältnisse ergibt sich wie folgt:

| **Eingang** | | **Ausgang** | |
|---|---|---|---|
| Kohlenstoffdioxid [g] | 845 kg | Wasser [g] | 692 kg |
| Wasserstoff [g] | 155 kg | Methan [g] | 308 kg |
| | 1.000 kg | | 1.000 kg |

Die Reaktion der Stoffumwandlung ist stark exotherm. Die thermische Energie wird über die Umwandlung des entstehenden Wassers zu Wasserdampf abgeleitet. Energetisch betrachtet, wird die chemische Energie aus dem Wasserstoff und dem Kohlenstoffdioxid in chemische Energie des Methans umgesetzt. Dabei entstehen Verluste durch Abgabe von thermischer Energie. Der eingesetzte Katalysator ist in der Regel nicht besonders aktiv. Verbesserte Katalysatoreigenschaften werden durch Zuführung von mechanischer und thermischer Energie erzielt.

In einem weiteren Ausführungsbeispiel sind nachfolgend stöchiometrische Berechnungen für ein theoretisches Verhältnis der Stoffströme bei Ethanol und Methanbildung zueinander auf Basis Einsatzgröße von 1.000kg Kohlenstoffdioxid dargestellt.

### Dampferzeugung

| **Eingang** | | **Ausgang** | |
|---|---|---|---|
| Sauerstoff [g] | 1.090 464 kg | Kohlenstoffdioxid [g] | 1.4992.013 kg |
| Kohlenstoff [s] | 409 549 kg | | |
| | 1.4992.013 kg | | 2.0131.499 kg |

### Elektrolyse

| **Eingang** | | **Ausgang** | |
|---|---|---|---|
| Wasser [I] | 1.227 648 kg | Wasserstoff [g] | 137 184 kg |
| | | Sauerstoff [g] | 1.090 464 kg |
| | 1.227 648 kg | | 1.227 648 kg |

### Ethanolherstellung

| **Eingang** | | **Ausgang** | |
|---|---|---|---|
| Glucose [s] | 2.046 kg | Kohlenstoffdioxid [g] | 1.000 kg |
| | | Ethylalkohol [I] | 1.046 kg |
| | 2.046 kg | | 2.046 kg |

### Methansynthese

| **Eingang** | | **Ausgang** | |
|---|---|---|---|
| Kohlenstoffdioxid [g] | 1.000 kg | Wasser [g] | 409 819 kg |
| Wasserstoff [g] | 137 184 kg | Methan [g] | 728 365 kg |
| | 1.137 184 kg | | 1.137 184 kg |

In einem weiteren Ausführungsbeispiel sind die berechneten Stoffströme im System für die Bildung von Ethanol und Methan in der Tab.2 dargestellt.

**Tab. 2**

| Nr | Stoff | Summenformel | Mittlere Molare Masse | Aggregatzustand | H₂ | N₂ | O₂ | Ar | CO₂ | C₆H₅OH | CH₄ | C₂H₆O | H₂O | sonst. |
|---|---|---|---|---|---|---|---|---|---|---|---|---|---|---|
| | | | [g/mol] | | [mol %] | [mol %] | [mol %] | [mol %] | [mol %] | [mol %] | [mol %] | [mol %] | [mol %] | [mol %] |
| 01 | Kohlenstoff, rein | C | 12,01 | s | --- | --- | --- | --- | --- | --- | --- | --- | --- | 100 |
| 02 | Luft, trocken | N₂,O₂, CO₂,... | 28,95 | g | --- | 78,08 | 20,94 | 0,93 | 0,03 | --- | --- | --- | --- | 0,002 |
| 03 | Wasser, vollentsalzt | H₂O | 18,02 | l | --- | --- | --- | --- | --- | --- | --- | --- | 100 | --- |
| 04 | Kohlenstoffdi oxid | CO₂ | 44,01 | g | --- | --- | --- | --- | 100 | --- | --- | --- | --- | --- |
| 05 | Wasser | H₂O | 18,02 | l | --- | --- | --- | --- | --- | --- | --- | --- | 100 | --- |
| 06 | Wasser | H₂O | 18,02 | g | --- | --- | --- | --- | --- | --- | --- | --- | 100 | --- |
| 07 | Sauerstoff | O₂ | 15,99 | g | --- | --- | 100 | --- | --- | --- | --- | --- | --- | --- |
| 08 | Wasser | H₂O | 18,02 | l | --- | --- | --- | --- | --- | --- | --- | --- | 100 | --- |
| 09 | Wasserstoff | H₂ | 1,01 | g | 100 | --- | --- | --- | --- | --- | --- | --- | --- | --- |
| 10 | Glucose | C₆H₁₂O₆ | 180,15 | s | --- | --- | --- | --- | --- | 100 | --- | --- | --- | --- |
| 11 | Kohlenstoffdi oxid | CO₂ | 44,01 | g | --- | --- | --- | --- | 100 | --- | --- | --- | --- | --- |
| 12 | Ethylalkohol | C₂H₆O | 46,07 | l | --- | --- | --- | --- | --- | --- | --- | 99,95 | --- | 0,05 |
| 13 | Abwasser | H₂O | 18,02 | l | --- | --- | --- | --- | --- | --- | --- | --- | 70 | 30 |
| 14 | Wasser | H₂O | 18,02 | g | --- | --- | --- | --- | --- | --- | --- | --- | 100 | --- |
| 15 | Methan | CH₄ | 16,04 | g | --- | --- | --- | --- | --- | --- | 99,95 | --- | --- | 0,05 |
| 16 | LuftSauerstoffGemisch | N₂, O₂, CO₂,... | 28,95 | g | --- | < 78,08 | > 20,94 | <0,93 | <0,03 | --- | --- | --- | --- | <0,002 |
| 17 | Wasser, vollentsalzt | H₂O | 18,02 | l | --- | --- | --- | --- | --- | --- | --- | --- | 100 | --- |

### Bezugszeichenliste

- 1: System
- 2: Vorrichtung zur biologischen Herstellung von Ethanol
- 3: Vorrichtung zur chemischen Synthese eines Kohlenwasserstoffes,
- 4: Ethanol
- 5: Methanol oder Methan
- 6: Kohlenhydrate
- 7: Wasser
- 8: Kohlenstoffdioxid
- 9: Prozessabwässer
- 10: Wasserstoff
- 11: Wasser als Produkt der chemischen Synthese des Kohlenwasserstoffs
- 12: Vorrichtung zur Dampferzeugung
- 13: Sauerstoff
- 14: Wasser
- 15: Kondensat
- 16: Kohlenstoffhaltige Verbindungen
- 17: Stickoxidhaltige Abgase
- 18: Vorrichtung zur Generation von Wasserstoff

## Patentansprüche

1. System (1) zur Erzeugung von aliphatischen Alkoholen, umfassend:
- zumindest eine erste Vorrichtung (2) zur biologischen Herstellung von Ethanol (4), wobei Kohlenstoffdioxid gebildet wird und
- zumindest eine zweite Vorrichtung (3) zur chemischen Umsetzung des Kohlenstoffdioxids (8) zu Methanol oder Methan,
wobei die zweite Vorrichtung (3) zur chemischen Synthese von Methanol oder Methan (5) der ersten Vorrichtung (2) zur biologischen Herstellung des Ethanols (4) nachgeschaltet ist, wobei
- das System (1) eine zweite Verbindungsleitung von der zweiten Vorrichtung (3) zur ersten Vorrichtung (2) aufweist, die es ermöglicht, in der zweiten Vorrichtung (3) gebildetes Wasser (11) in die erste Vorrichtung (2) einzuleiten, wobei das in der zweiten Vorrichtung (3) gebildete Wasser (11) zum Anmaischen verwendet wird,
- weiterhin zumindest eine Vorrichtung zur Dampferzeugung (12) und eine dritte Verbindungsleitung umfasst, wobei die dritte Verbindungsleitung zwischen der Vorrichtung zur Dampferzeugung (12) und der ersten Vorrichtung (2) angeordnet ist und es ermöglicht, den in der Vorrichtung zur Dampferzeugung (12) gebildeten Dampf (7) in die erste Vorrichtung (2) einzuleiten und wobei
- das System (1) eine vierte Verbindungsleitung von der ersten Vorrichtung (2) zur Vorrichtung zur Dampferzeugung (12) aufweist, die es ermöglicht, ein in der ersten Vorrichtung (2) gebildetes Kondensat (15) in die Vorrichtung zur Dampferzeugung (12) einzuleiten.

2. System nach Anspruch 1, **dadurch gekennzeichnet, dass** das System (1) eine erste Verbindungsleitung von der ersten Vorrichtung (2) zur zweiten Vorrichtung (3) aufweist, die es ermöglicht, ein in der ersten Vorrichtung (2) gebildetes Kohlenstoffdioxid (8) in die zweite Vorrichtung (3) einzuleiten.

3. System nach einem der Ansprüche 1 oder 2 weiterhin umfassend zumindest eine Vorrichtung zur Generation von Wasserstoff (16), welche eine fünfte Verbindungsleitung zur zweiten Vorrichtung (3) aufweist, die es ermöglicht, den Wasserstoff (10) in die zweite Vorrichtung (3) einzubringen.

4. System nach einem der vorhergehenden Ansprüche, **dadurch gekennzeichnet, dass** die Vorrichtung zur Generation von Wasserstoff (16) als Elektrolysevorrichtung ausgebildet ist.

5. System nach einem der vorhergehenden Ansprüche, **dadurch gekennzeichnet, dass** das System (1) eine sechste Verbindungsleitung aufweist, die es ermöglicht, den in der Elektrolysevorrichtung (16) gebildeten Sauerstoff (13) in die Vorrichtung zur Dampferzeugung (12) einzuleiten.

6. Verfahren zur Herstellung von aliphatischen Alkoholen, umfassend:
- Herstellung eines ersten aliphatischen Alkohols (4) mittels eines biologischen Verfahrens, wobei Kohlenstoffdioxid (8) gebildet wird, wobei der erste aliphatische Alkohol (4) Ethanol ist,
- Umsetzung des Kohlenstoffdioxids (8) zu Methanol oder Methan (5) mittels chemischer, katalytischer Synthese,
wobei die Umsetzung des Kohlenstoffdioxids (8) der Herstellung des ersten aliphatischen Alkohols (4) nachgeschaltet und örtlich getrennt ist und die Herstellung des ersten aliphatischen Alkohols (4) und die Umsetzung des Kohlenstoffdioxids (8) derart verbunden sind, dass das Kohlenstoffdioxid (8) als Produkt der Herstellung des ersten aliphatischen Alkohols (4) zumindest teilweise als Edukt zur Herstellung des Methanols oder Methans (5) verwendet wird, wobei bei der Umsetzung des Kohlenstoffdioxids (8) zu Methanol oder Methan gebildetes Wasser (11) bei der Herstellung des Ethanols (4) zum Anmaischen verwendet wird, und weiterhin umfassend ein Verfahren zur Dampferzeugung, welches derart mit der Herstellung des Ethanols (4) verbunden ist, dass der im Verfahren zur Dampferzeugung generierte Dampf (7) zum Eintrag thermischer Energie für die Herstellung des Ethanols (4) verwendet wird und ein bei der Herstellung des Ethanols (4) gebildetes Kondensat (15) dem Verfahren zur Dampferzeugung (12) zugeführt wird.

7. Verfahren nach Anspruch 6 weiterhin umfassend ein Verfahren zur Generation von Wasserstoff, welches derart mit der Herstellung des Methanols oder Methans (5) verbunden ist, dass der generierte Wasserstoff (10) als Edukt für die chemische Synthese des Methanols oder Methans (5) dient, wobei das Verfahren zur Generation von Wasserstoff ein auf der Elektrolyse von Wasser (14) basierendes Verfahren ist, bei dem neben Wasserstoff (10) durch die Elektrolyse auch Sauerstoff (13) gebildet wird, welcher für das Verfahren zur Dampferzeugung genutzt wird, wobei das Verfahren ausgewählt ist aus alkalischer Elektrolyse und PEM-Elektrolyse.

8. Verfahren nach einem der Ansprüche 6 oder 7, **dadurch gekennzeichnet, dass** bei der Herstellung des Methanols oder Methans (5) Wasser (11) gebildet wird, welches für das Verfahren zur Generation von Wasserstoff eingesetzt wird.

## Claims

1. A system (1) for the production of aliphatic alcohols, comprising:
- at least one first device (2) for the biological production of ethanol (4), wherein carbon dioxide is formed, and
- at least one second device (3) for chemically reacting the carbon dioxide (8) to form methanol or methane,
wherein the second device (3) for the chemical synthesis of methanol or methane (5) is downstream of the first device (2) for the biological production of ethanol (4), wherein
- the system (1) comprises a second connecting line from the second device (3) to the first device (2) which allows water (11) formed in the second device (3) to be introduced into the first device (2), wherein the water (11) formed in the second device (3) is used for mashing,
- further comprising at least one device (12) for producing steam and a third connecting line, wherein the third connecting line is disposed between the steam production device (12) and the first device (2) and allows the steam (7) formed in the steam production device (12) to be introduced into the first device (2), and wherein
- the system (1) comprises a fourth connecting line from the first device (2) to the steam production device (12) which allows a condensate (15) formed in the first device (2) to be introduced into the steam production device (12).

2. The system as claimed in claim 1, **characterized in that** the system (1) comprises a first connecting line from the first device (2) to the second device (3) which allows the carbon dioxide (8) formed in the first device (2) to be introduced into the second device (3).

3. The system as claimed in claim 1 or claim 2, further comprising at least one device for generating hydrogen (16), which comprises a fifth connecting line to the second device (3) which allows the hydrogen (10) to be introduced into the second device (3).

4. The system as claimed in one of the preceding claims, **characterized in that** the device for the generation of hydrogen (16) is configured as an electrolysis device.

5. The system as claimed in one of the preceding claims, **characterized in that** the system (1) comprises a sixth connecting line which allows the oxygen (13) formed in the electrolysis device (16) to be introduced into the steam production device (12).

6. A process for the production of aliphatic alcohols, comprising:
- producing a first aliphatic alcohol (4) by means of a biological process, wherein carbon dioxide (8) is formed, wherein the first aliphatic alcohol (4) is ethanol,
- reacting the carbon dioxide (8) to form methanol or methane (5) by means of chemical, catalytic synthesis,
wherein the carbon dioxide (8) reaction is downstream of the production of the first aliphatic alcohol (4) and separated therefrom in space and the production of the first aliphatic alcohol (4) and the reaction of the carbon dioxide (8) are connected in a manner such that the carbon dioxide (8) is used as a product of the production of the first aliphatic alcohol (4) at least in part as an educt for the production of methanol or methane (5), wherein water (11) formed during the reaction of the carbon dioxide (8) to methanol or methane is used in the production of ethanol (4) for mashing, and further
comprising a steam production process which is linked to the production of the ethanol (4) in a manner such that the steam (7) generated during the steam production process is used to input thermal energy for the production of ethanol (4) and a condensate (15) formed during the production of the ethanol (4) is supplied to the process (12) for the production of steam.

7. The process as claimed in claim 6, further comprising a process for the generation of hydrogen which is linked to the production of the methanol or methane (5) in a manner such that the hydrogen (10) generated acts as an educt for the chemical synthesis of methanol or methane (5), wherein the process for the generation of hydrogen is a process based on the electrolysis of water (14), in which in addition to hydrogen (10), oxygen (13) is also formed by the electrolysis which is used for the steam production process, wherein the process is selected from alkaline electrolysis and PEM electrolysis.

8. The process as claimed in claim 6 or claim 7, **characterized in that** during the production of methanol or methane (5), water (11) is formed which is employed for the process for the generation of hydrogen.

## Revendications

1. Système (1) de fabrication d'alcools aliphatiques, comprenant :
- au moins un premier dispositif (2) de production biologique d'éthanol (4) impliquant la formation de dioxyde de carbone, et
- au moins un deuxième dispositif (3) destiné à soumettre le dioxyde de carbone (8) à une réaction chimique le transformant en méthanol ou méthane,
le deuxième dispositif (3) de synthèse chimique de méthanol ou de méthane (5) étant situé en aval du premier dispositif (2) de production biologique de l'éthanol (4),
- le système (1) comportant une deuxième conduite de liaison allant du deuxième dispositif (3) au premier dispositif (2), laquelle permet d'introduire dans le premier dispositif (2) de l'eau (11) formée dans le deuxième dispositif (3), ladite eau (11) formée dans le deuxième dispositif (3) étant utilisée pour préparer le mélange à fermenter,
- comprenant en outre au moins un dispositif de génération de vapeur (12) et une troisième conduite de liaison, la troisième conduite de liaison étant disposée entre le dispositif de génération de vapeur (12) et le premier dispositif (2) et permettant d'introduire la vapeur (7), formée dans le dispositif de génération de vapeur (12), dans le premier dispositif (2), et
- le système (1) comportant une quatrième conduite de liaison allant du premier dispositif (2) au dispositif de génération de vapeur (12) et permettant d'introduire un condensat (15), formé dans le premier dispositif (2), dans le dispositif de génération de vapeur (12).

2. Système selon la revendication 1, **caractérisé en ce que** le système (1) comporte une première conduite de liaison allant du premier dispositif (2) au deuxième dispositif (3) et permettant d'introduire du dioxyde de carbone (8), formé dans le premier dispositif (2), dans le deuxième dispositif (3).

3. Système selon l'une des revendications 1 ou 2, comprenant en outre au moins un dispositif de génération d'hydrogène (16) comportant une cinquième conduite de liaison vers le deuxième dispositif (3) laquelle permet d'introduire l'hydrogène (10) dans le deuxième dispositif (3).

4. Système selon l'une des revendications précédentes, **caractérisé en ce que** le dispositif de génération d'hydrogène (16) est réalisé sous forme d'un dispositif d'électrolyse.

5. Système selon l'une des revendications précédentes, **caractérisé en ce que** le système (1) comporte une sixième conduite de liaison permettant d'introduire l'oxygène (13), formé dans le dispositif d'électrolyse (16), dans le dispositif de génération de vapeur (12).

6. Procédé de fabrication d'alcools aliphatiques, comprenant :
- la production d'un premier alcool aliphatique (4) au moyen d'un procédé biologique impliquant la formation de dioxyde de carbone (8), le premier alcool aliphatique (4) étant l'éthanol,
- la mise en oeuvre d'une réaction transformant le dioxyde de carbone (8), au moyen d'une synthèse chimique catalysée, en méthanol ou méthane (5),
la réaction transformant le dioxyde de carbone (8) étant mise en oeuvre dans un lieu séparé qui est situé en aval de la production du premier alcool aliphatique (4), la production du premier alcool aliphatique (4) et la transformation du dioxyde de carbone (8) étant reliées de manière à ce que le dioxyde de carbone (8), issu de la production du premier alcool aliphatique (4), soit au moins partiellement mis en oeuvre en tant que réactif entrant dans la préparation du méthanol ou du méthane (5) tout en utilisant l'eau (11), laquelle se forme lors de la transformation du dioxyde de carbone (8) en méthanol ou méthane, lors de la production de l'éthanol (4) pour préparer le mélange à fermenter, et
comprenant, en outre, un procédé de génération de vapeur qui est relié à la production de l'éthanol (4) de manière à ce que la vapeur (7), générée dans le procédé de génération de vapeur, soit utilisée pour apporter de l'énergie thermique destinée à la production de l'éthanol (4) et qu'un condensat (15), formé lors de la production de l'éthanol (4), soit introduit dans le procédé de génération de vapeur (12).

7. Procédé selon la revendication 6, comprenant en outre un procédé de génération d'hydrogène lequel est relié à la préparation du méthanol ou de méthane (5) de manière à ce que l'hydrogène (10) serve, suite à sa génération, de réactif entrant dans la synthèse chimique du méthanol ou du méthane (5), le procédé de génération d'hydrogène étant un procédé basé sur l'électrolyse d'eau (14) dans lequel l'électrolyse conduit à la formation d'hydrogène (10) mais également d'oxygène (13), ce dernier étant mis en oeuvre dans le procédé de génération de vapeur, le procédé étant choisi parmi l'électrolyse alcaline et l'électrolyse PEM.

8. Procédé selon l'une des revendications 6 ou 7, **caractérisé en ce que** la préparation du méthanol ou du méthane (5) implique la formation d'eau (11) laquelle est mise en oeuvre dans le procédé de génération d'hydrogène.
